# EUROPÄISCHE PATENTANMELDUNG

(11) **EP 0 737 439 A2**
(43) Veröffentlichungstag der Anmeldung: **16.10.1996**
(21) Anmeldenummer: 96105248.7
(22) Anmeldetag: 02.04.1996
(51) Int. Cl.: A61B 6/08, A61B 6/12

(54) **Vorrichtung zur Ortung von Konkrementen in Körpern von Lebewesen**

(30) Priorität: 08.04.1995 DE 19513400
(71) Anmelder: Dornier Medizintechnik GmbH, 81663 München (DE)
(72) Erfinder: Ueberle, Friedrich, Dr., 82205 Gilching (DE); Köninger, Arne, 86934 Ludenhausen (DE)
(74) Vertreter: Hummel, Adam

(57) **Zusammenfassung**

Die Vorrichtung zur Ortung von Konkrementen in Körpern von Lebewesen, im Hinblick auf eine Positionierung einer Therapieanordnung, insbesondere eines Lithotripters, weist eine erste Anordnung (2) auf, die eine Ortungsröntgenstrahlung in einer im wesentlichen senkrechten Richtung erzeugt und eine zweite Anordnung (3), die eine Ortungsröntgenstrahlung in einer unter einem Winkel zur senkrechten Richtung der ersten Anordnung verlaufenden Richtung erzeugt sowie eine zusätzliche Anordnung zur optischen Positionsmarkierung (8) eines Oberflächenbereichs des Körpers (1). Diese Markierung (8) besteht aus einem ersten Material, das durch einen auftreffenden Lichtstrahl (4) abtastbar ist und aus einem zweiten Material, das durch eine auftreffende Röntgenstrahlung abtastbar ist.

## Beschreibung

Die vorliegende Erfindung betrifft eine Vorrichtung zur Ortung von Konkrementen in Körpern von Lebewesen im Hinblick auf eine Positionierung einer Therapieanordnung, insbesondere eines Lithotripters, nach dem Oberbegriff des Anspruchs 1.

Bei der Behandlung von Patienten mit Nieren-, Gallen- und Gallenblasensteinen ist eine genaue Ortung der Lage dieser Konkremente äußerst wichtig. Die Ortung erfolgt üblicherweise mittels Röntgenstrahlung, wobei eine senkrecht auf den Körper auftreffende und diesen durchleuchtende Röntgenstrahlung (AP-Strahlung) mit einer schräg auf denselben Oberflächenbereich des Körpers auftreffenden und diesen durchleuchtenden Röntgenstrahlung (CC-Strahlung) kombiniert wird. Bei beiden Röntgenstrahlung-Durchleuchtungsvorgängen können laterale Fehlpositionen, d.h. Lagen der Konkremente quer zur Ortungs- und zur Patientenachse Y festgestellt werden. Bei der Ortung mittels der AP-Strahlung kann auch eine Fehlposition in Patientenlängsrichtung X ermittelt werden, während bei der schräg auftreffenden CC-Strahlung nicht zu unterscheiden ist, ob das Konkrement in X-Richtung oder in Z-Richtung ausgewandert ist. Aus diesem Grund wird bei der Röntgenortung üblicherweise der Körper zunächst durch die senkrecht auftreffende AP-Strahlung durchleuchtet und das Konkrement in X-Richtung und Y-Richtung richtig positioniert, beispielsweise anhand einer entsprechenden Bildschirmmarke. Bei der anschließenden Ortung mittels der schräg auftreffenden CC-Strahlung kann die richtige Lage des Konkrementes in Z-Richtung nur dann genau ermittelt werden, wenn sich der Patient zwischenzeitlich nicht mehr bewegt hat und demzufolge das Konkrement seine Lage bezüglich der vorhergehenden Ortung durch die AP-Strahlung nicht mehr verändert hat.

Bei einigen Lithotriptern muß zur Ortung mittels der AP-Strahlung der Therapiekopf abgekoppelt werden, so daß bei jeder Röntgenortung eine Behandlungsunterbrechung erforderlich wird, während der die Gefahr einer Bewegung des Patienten mit dieser innewohnender Lageänderung der Konkremente besteht.

Die DE-OS 42 33 632 beschreibt eine Vorrichtung zum Zertrümmern von Körperkonkrementen, die einen Therapieschallsender aufweist, der therapeutisch wirksame akustische Wellen erzeugt, die auf das zu zerstörende Konkrement fokussierbar sind, eine Ortungseinrichtung aufweist, die eine Röntgen- und/oder eine Ultraschalleinrichtung aufweist, die die Lage des zu zerstörenden Konkrementes erfaßt und die zur Ausrichtung des Fokusbereiches der therapeutisch wirksamen akustischen Wellen auf das Konkrement vor Beginn des Behandlungsvorganges dient, sowie eine Nachführeinrichtung aufweist, die bei einer Verlagerung des Konkrements den Fokusbereich der therapeutisch wirksamen akustischen Wellen durch eine Relativbewegung Fokusbereich/Konkrement nachführt. Zu diesem Zweck weist die Ortungseinrichtung eine weitere Sensoreinheit in Form einer Auflagekugel auf, die an einem Arm um eine Achse schwenkbar gelagert ist, wobei die Kugel auf einen Bereich des Körpers aufgelegt wird und die Drehung des Arms um die Anlenkachse durch einen Aufnehmer erfaßt wird, dessen Ausgangssignal einer Schaltung zugeführt wird, die den Fokusbereich des Therapieschallsenders verändert. Eine Veränderung der Lage des Patienten ist bei dieser bekannten Vorrichtung nicht vorgesehen.

Die DE-OS 41 20 074 bezieht sich auf ein Verfahren und eine Vorrichtung zum Steuern der Position des Körpers eines Patienten, der mit einem Lithotripter behandelt wird, wobei die Vorrichtung eine Einrichtung aufweist, um ein Lichtbild auf eine bestimmte Zone des Patientenkörpers zu projizieren so wie dieser seine korrekte Lage zur Behandlung eingenommen hat.

Anschließend wird eine Markierung des auf den Körper projizierten Bildes mittels eines Markierungsschreibers angebracht und während der therapeutischen Behandlung beobachtet, so daß jede Abweichung der aufgemalten Markierung vom projizierten Lichtbild vom behandelnden Arzt festgestellt werden kann. Im Falle einer Lageabweichung der Markierung vom Bild ist die medizinische Behandlung zu unterbrechen und der Patient aufzufordern, die Position seines Körpers dergestalt zu ändern, daß die Markierung wieder in Übereinstimmung mit dem projizierten Bild gelangt (Spalte 4, Zeilen 30 bis 51).

Aufgabe der vorliegenden Erfindung ist es, das Ortungsverfahren für die Konkremente zu vereinfachen und zu verbessern und dafür zu sorgen, daß die Behandlung des Patienten auch bei einer Lageverschiebung der Konkremente während deren Zertrümmerung nicht unterbrochen werden muß.

Ausgehend von einer Vorrichtung der eingangs näher genannten Art, erfolgt die Lösung dieser Aufgabe mit den im kennzeichnenden Teil des Anspruchs 1 angegebenen Merkmalen; vorteilhafte Ausgestaltungen sind in den Unteransprüchen beschrieben.

Die Erfindung weist also den Vorteil auf, daß durch die zusätzliche Positionsmarkierung und die Einrichtung zur Beurteilung der Lageverschiebung der Markierung und damit des Konkrementes ein manuelles oder automatisches Nachpositionieren des Patienten ermöglicht wird, der dazu entsprechend gelagert wird, so daß die Behandlung nicht unterbrochen werden muß. Die Genauigkeit der Ortung wird dadurch erhöht, daß gleichzeitig sowohl eine Röntgenstrahlungortung als auch eine optische Ortung erfolgt, da die aufgebrachte Markierung sowohl von der Röntgenstrahlung als auch von der optischen Strahlung abgetastet werden kann.

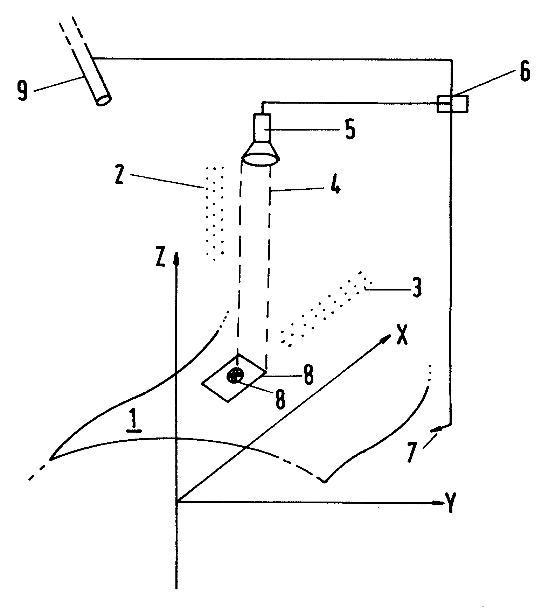

Im folgenden wird die Erfindung anhand der Zeichnung näher erläutert, in der ein vorteilhaftes Ausführungsbeispiel dargestellt ist. Die einzige Figur zeigt schematisch einen Teil einer mit einem Lithotripter zu behandelnden Körperoberfläche, mit einer daran angebrachten Markierung und die auf diese Markierung auftreffenden Strahlen.

In der einzigen Figur ist mit 1 schematisch ein Körper eines Lebewesens bezeichnet, in dem ein (nicht dargestelltes) Konkrement, beispielsweise ein Gallenblasenstein, durch die übliche Stoßwellentherapie zertrümmert werden soll. Zur Ermittlung der richtigen Lage des Konkrementes im Körper wird der auf einer Patientenliege ruhende Körper 1 zuerst mit einer senkrecht auftreffenden Röntgenstrahlung 2 durchleuchtet und anschließend mit einer schräg auftreffenden Röntgenstrahlung 3, um dadurch das Konkrement im Koordinatensystem X, Y, Z für die nachfolgende Stoßwellentherapie zu orten.

Nach der Ortung durch die beiden mit 2 und 3 angedeuteten Röntgenstrahlungen wird die Oberfläche des Körpers erfindungsgemäß mit einer zusätzlichen Positionsmarkierung 8, 8' versehen, die aus unterschiedlichen Materialien besteht, von denen z.B. das Material 8 für eine auftreffende optische Strahlung 4, die von einer Lichtquelle 5 abgegeben wird, empfindlich ist, z.B. reflektierend, und das Material 8' für eine auftreffende Röntgenstrahlung empfindlich ist, d.h. z.B. aus Metall besteht, welches auf dem der Röntgenstrahlung zugeordneten Röntgenbildverstärker eine entsprechende Markierung erzeugt. Vorteilhafterweise wird z.B. eine optische Anordnung 9 verwendet, die aus einer fernrohrähnlichen Einrichtung bestehen kann, um eine Abweichung, d.h. Verschiebung, der zusätzlichen Markierung 8, 8' aus ihrer Sollage zu beobachten. Sobald eine derartige Verschiebung auftritt, kann über eine Steueranordnung 6, die über eine Leitung 7 mit der (nicht dargestellten) Positionieranordnung für die Patientenliege verbunden ist, letzterer derart verstellt werden, daß die zusätzliche Markierung 8, 8' ihre ursprüngliche Lage wieder einnimmt, so daß eine ungehinderte weitere Therapie des Konkrementes ohne Unterbrechung möglich ist.

Es ist selbstverständlich auch möglich, zwei Markierungen am Körper 1 des Patienten einzusetzen, die dann derart an der Körperoberfläche angebracht werden, daß ihre Ebenen senkrecht aufeinanderstehen; bei gleichzeitiger Beobachtung dieser beiden Markierungen durch zwei Detektoren kann eine dreidimensionale Auswertung von Positionsänderungen erfolgen, anstelle einer zweidimensionalen Lageänderung in der X,Y-Ebene, wie sie bei der in der einzigen Figur dargestellten schematischen Anordnung ermöglicht wird.

Ferner ist es auch möglich, anstelle einer Veränderung der Patientenliege, sei es in der X,Y-Ebene oder im dreidimensionalen Raum, eine Nachpositionierung des Stoßwellentherapiegerätes vorzunehmen, bei einer Lageveränderung des Körpers 1 und damit Auswandern des zu behandelnden Konkrementes aus dem ursprünglich eingestellten Fokus des Stoßwellentherapiegerätes.

## Patentansprüche

1. Vorrichtung zur Ortung von Konkrementen in Körpern von Lebewesen, im Hinblick auf eine Positionierung einer Therapieanordnung, insbesondere eines Lithotripters, mit einer ersten Anordnung, die eine Ortungsröntgenstrahlung in einer im wesentlichen senkrechten Richtung zur Körperoberfläche und zur Körperlängsachse erzeugt und mit einer zweiten Anordnung, die eine Ortungsröntgenstrahlung in einer unter einem Winkel zur senkrechten Richtung der ersten Anordnung und zur Körperlängsachse verlaufenden Richtung erzeugt, und mit einer zusätzlichen Anordnung zur optischen Positionsmarkierung eines Oberflächenbereichs des Körpers, die eine Lichtquelle und eine Markierung sowie eine Einrichtung zur Beurteilung der Lage der Markierung aufweist, dadurch gekennzeichnet, daß die Markierung aus einem ersten Teil (8), der durch einen auftreffenden Lichtstrahl (4) abtastbar ist, und aus einem zweiten Teil (8') besteht, der durch die auftreffende Röntgenstrahlung (2, 3) abtastbar ist.

2. Vorrichtung zur Ortung nach Anspruch 1, dadurch gekennzeichnet, daß der erste Teil (8) der Markierung einen Spiegel enthält und daß der zweite Teil (8') eine Metallfolie enthält.

3. Vorrichtung nach einem der vorhergehenden Ansprüche, dadurch gekennzeichnet, daß die Einrichtung (9) zur Beurteilung der Lage der Markierung eine optische Einrichtung in Form eines Fernrohrs aufweist sowie einen Positionsmelder (6) für eine Lageverschiebung der Markierung und damit des Körpers (1).

4. Vorrichtung nach Anspruch 3, dadurch gekennzeichnet, daß der Positionsmelder (6) mit einer Einrichtung zur automatischen Lagekorrektur des Körpers (1) verbunden ist.

5. Vorrichtung nach einem der vorhergehenden Ansprüche, dadurch gekennzeichnet, daß der Körper (1) mit zwei Markierungen versehen ist, die in senkrecht zueinander verlaufenden Ebenen angeordnet sind und getrennt beobachtbar sind, so daß eine dreidimensionale Lageverschiebung des mit den beiden Markierungen versehenen Körpers (1) möglich ist.
